# EUROPEAN PATENT APPLICATION

(11) **EP 4 793 283 A1**
(43) Date of publication of application: **19.08.2026**
(21) Application number: 25158171.6
(22) Date of filing: 17.02.2025
(51) Int. Cl.: C07K 14/47, A61K 38/00, C07K 7/64, G01N 33/68

(54) **PEPTIDES BLOCKING AMYLOID FIBRIL GROWTH**

(71) Applicant: Ustav Organicke Chemie a Biochemie AV CR, v.v.i., 16000 Praha 6 (CZ)
(72) Inventor: Galkin, Maksym, Kyiv (UA); Shvadchak, Volodymyr, Kalush (UA)
(74) Representative: Harber IP s.r.o.

(57) **Abstract**

The present invention belongs the field of biomedicine, namely, treating and preventing Parkinson's and Alzheimer's diseases. These diseases are characterized by the presence of amyloid fibrils that drive the pathology progression in the brains of affected individuals. The invention discloses peptides that block ends of amyloid fibrils and stop their growth.

## Description

### Technical field

The present invention relates to peptides that block amyloid fibril growth. Such peptides can be used as therapeutic agents for treatment of Alzheimers and Parkinson's diseases.

### Background art

Alzheimer's disease (AD) and Parkinson's disease (PD) are the most common neurodegenerative diseases worldwide. Overall, AD affects more than 4 % of people over the age of 60 years, while PD affects 1% of people of this age. Almost 20% of people older than 80 years old is expected to suffer from either of these conditions (Kalia & Lang, Lancet, 2015, doi: 10.1016/S0140-6736(14)61393-3).

The mechanism of these diseases involves the selective death of neurons that is caused by accumulation of misfolded protein in the form of amyloid fibrils. These fibrils predominantly consist of amyloid beta and tau in case of AD, and alpha-synuclein in the case of PD.

To date these neurogenerative conditions are considered to be uncurable and only symptomatic treatment is available. It mostly relies on acetylcholinesterase inhibitors (AD) and dopaminergic drugs (PD) to reduce the symptoms. However, these treatment options cannot halt the progression of the disease, offering only a temporary solution.

Currently, the mechanism by which amyloid protein deposits cause neuronal death is poorly understood. The proposed mechanisms include direct damage to the membranes or involvement of neuroinflammation that lead to neuron death. However, majority of the existing evidence points to correlation between the amount of the deposits and the extent of neurodegeneration. Therefore, inhibition of amyloid fibril growth and their subsequent spreading is a promising approach for the disease treatment (Tysnes & Storstein, J Neural Transm, 2017, doi: 10.1007/s00702-017-1686-y).

The involvement of pathological protein deposition in the progression of PD is additionally highlighted by the fact that individuals with either additional copies of gene coding αSyn (Chartier-Harlin et al., Lancet, 2004, doi: 10.1016/S0140-6736(04)17103-1) or its mutations that increase αSyn aggregation propensity (Ruf et al., ACS Chem Neursci, 2019, doi: 10.1021/acschemneuro.8b00579) are at a higher risk of the early development of PD. Moreover, peripheral injection of αSyn fibrils induces aggregation of αSyn in neurons in vivo (Jucker & Walker, Nat Neurosci, 2018, doi: 10.1038/s41593-018-0238-6) suggesting that aggregation of αSyn can spread along neuron pathways via a prion-like mechanism (Hijaz & Volpicelli-Daley, Mol Neurodegener, 2020, doi: 10.1186/s13024-020-00368-6).

Similarly, individuals with enhanced activity of gamma-secretase, an enzyme involved in the production of Aβ, have significantly higher chances of developing AD.

Amyloid fibrils of αSyn and Aβ are composed of hundreds of monomers in β-sheet conformation connected by intermolecular hydrogen bonds making them resilient to proteolytic degradation. Amyloid fibrils reach several microns in length that correspond to thousands of monomers and contain two monomers per cross-section.

Amyloid fibril formation includes three key processes: primary nucleation, elongation, fibril-dependent nucleation. The primary nucleation is the formation of proto fibrils directly from monomers. This process in known to be relatively slow, usually taking several days even at relatively high concentrations of Aβ or αSyn *in vitro.* Fibril elongation is monomer attachment to fibril end and adoption of amyloid conformation, increasing the fibril length in a one-by-one manner. Elongation is significantly faster than the primary nucleation and it is the process by which the vast majority of fibril mass is formed. When fibril becomes long enough, it can induce formation of new fibril ends (fibril growth centers) by breaking or other fibril-dependent nucleation processes. Such fibril-dependent secondary nucleation makes the fibril growth an autocatalytic process and leads to exponential increase of the amount of fibrillized protein.

One of the most prospective approaches to inhibit amyloid fibril growth is to block fibril ends that serve as growth centers. Unlike monomeric Aβ or αSyn, their amyloid fibrils have defined structure and their ends can serve as a molecular target, for development of specific inhibitor molecules. Fibrils contain hundreds to thousands of monomers, therefore the concentration of fibril ends is orders of magnitude lower than the concentration of monomeric protein (either αSyn or Aβ). Moreover, this approach does not interfere with the physiological function of monomeric αSyn or APP as it targets pathological species only.

There are a number of compounds that have been reported to inhibit amyloid formation to date. The most notable examples are several small molecules such as polyphenolic compounds (EGCG, quercetin), and anle138b (Wagner et al., Acta Neuropathol, 2013, doi: 10.1007/s00401-013-1114-9). However, their mechanism of action is based on either stabilization of monomeric protein or destabilization of the fibrils. For the inhibition, they require concentrations equal to or greater than those of the targeted species. Therefore, these inhibitors and their analogues are unable to provide the necessary effect in substechiometric concentrations. Additionally, small molecules have low specificity due to lack of well-defined molecular target.

Another class of existing inhibitors of αSyn fibrillization are natural and engineered proteins. Due to their large size, they are able to selectively recognize and block ends of amyloid fibrils. However, the most potent of them have molecular weight of 8 kDa (Priss, J Med Chem, 2021, doi: 10.1021/acs.jmedchem.1c00086) or 5 kDa (Murray, PNAS, 2022, doi: 10.1073/pnas.2206240119) and their practical application faces challenges such as delivery limitations across the blood-brain barrier (BBB).

### Disclosure of the Invention

The present invention provides peptides which are useful as drugs to treat Parkinson's and/or Alzheimer's diseases by suppressing the formation of amyloid protein deposits in the brain. They act as inhibitors of the growth of amyloid fibrils preventing the accumulation of the pathological amyloid deposits.

Object of the invention are cyclic peptides containing 17 to 35 amino acids and comprising a peptide sequence of general structure R¹-X¹-C-X²-X³X⁴X⁵X⁶X⁷-X⁸-KKK-X⁹-X¹⁰X¹¹X¹²X¹³X¹⁴-X¹⁵-C-X¹⁶ (SEQ ID NO. 1), wherein R¹ is selected from carboxyfluorescein residue, Ph₃C-CH₂-CO-, Ph₃P⁺(CH₂)₃-CO-, Ph₂N-(C₆H₄)-CO- and sulforhodamine residue attached to the -NH- group of the N-terminal amino acid; each of X³, X⁴, X⁵, X⁶, X⁷, X¹⁰, X¹¹, X¹², X¹³, X¹⁴ is independently selected from the amino acids V, I, A and T; each of X², X⁸, X⁹ and X¹⁵ independently represents 0 to 2 amino acids; and each of X¹ and X¹⁶ independently represents 0 to 6 amino acids; and wherein the two cysteines (C) are connected via a linker, thus forming a cycle.

The term "amino acid", unless further specified by the immediate context, refers to the naturally occuring amino acids. Namely, these amino acids are alanine (A), arginine (R), asparagine (N), aspartate (D), cysteine (C), glutamine (Q), glutamate (E), glycine (G), histidine (H), isoleucine (I), leucine (L), lysine (K), methionine (M), phenylalanine (F), proline (P), serine (S), threonine (T), tryptophan (W), tyrosine (Y), valine (V).

Preferably, X¹ is selected from K, KK, GK and WGK.

Preferably, X² is K or is not present.

Preferably, X⁸ is not present.

Preferably, X⁹ is WG, HG or is not present

Preferably, X¹⁵ is K or is not present.

Preferably, X¹⁶ is selected from K, E, RK, RKK, RRK, KRRK (SEQ ID NO. 19).

Particularly preferably, X² and X¹⁵ are the same.

In a preferred embodiment the peptide of the invention comprises the sequence R¹-X¹-C-X²-VATVA-X⁸-KKK-X⁹-VATVA-X¹⁵-C-X¹⁶ (SEQ ID NO. 2).

In a particularly prefered embodiment the peptide of the invention comprises the sequence R¹-K-C-K-VATVA-KKK-WG-VATVA-K-C-RRK (SEQ ID NO. 3). In this embodiment, it is further preferred when the linker connecting the two cysteines is m-xylenediyl.

Preferably, the linker connecting the two cysteins in the peptide of the invention is selected from disulfide bond, -CH₂-(C₆H₄)-CH₂-, -(CH₂)ₙ- wherein n=2-6, -CH₂CH₂OCH₂CH₂-, and bismaleimide linker. Preferably, the linker is selected from disulfide bond and m-xylenediyl.

Another aspect of the invention are the peptides of the invention for use as a medicament.

A further object of the invention are the peptides of the invention for use in the prevention or treatment of a disorder selected from Parkinson's and Alzheimer's diseases.

Yet another object of the invention is a pharmaceutical composition, which contains at least one peptide of the invention as an active ingredient and at least one pharmaceutically acceptable excipient.

Yet further object of the invention is *in vitro* method for reducing or inhibiting aggregation of αSyn, Aβ, or their mutants, comprising contacting amyloid fibrils with the peptide of the invention.

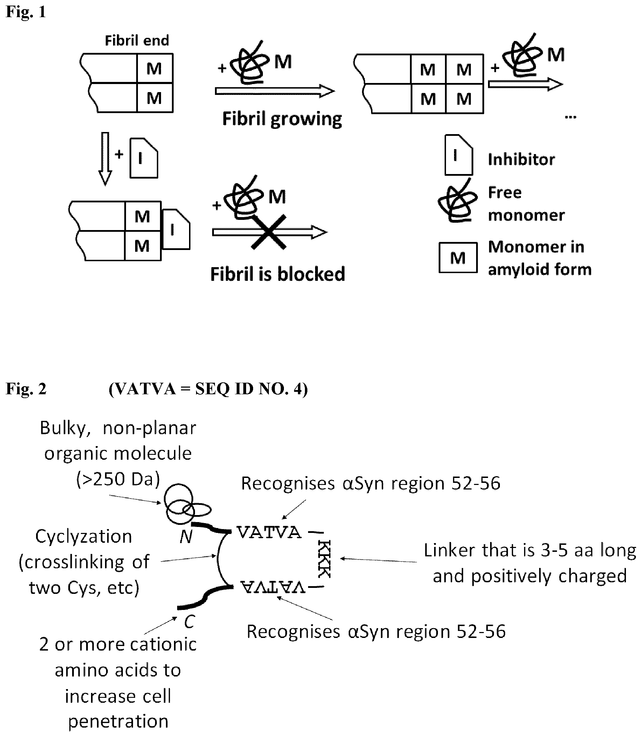

The peptides of the invention show affinity for binding to the end of amyloid fibril at the interface of two fibril filaments. They contain sequences mimicking the sequences of two αSyn monomers on the interface of two fibril filaments that provides tight and selective binding to the end of amyloid fibril. This is achieved by interacting simultaneously with two molecules of fibrillized protein (Fig 1). Simultaneous binding to two monomers increases the affinity and, therefore, provides stronger inhibition of fibril growth. The peptides of the invention also contain modification by introduction of non-planar bulky group R¹ containing several aromatic rings (with overall molecular mass 250 Da or higher). The bulky group due to its non-planar nature creates steric hindrance for binding of monomeric amyloidogenic protein to the fibril end occupied with peptide. Therefore, the peptides block ends of amyloid fibrils, restricting binding of monomeric protein and inhibiting amyloid fibril growth (Fig 1). The disclosed approach of combining an amyloid-binding sequence with a bulky group is primarily designed to target αSyn fibril growth, however it was also proved to be effective for Aβ fibrils.

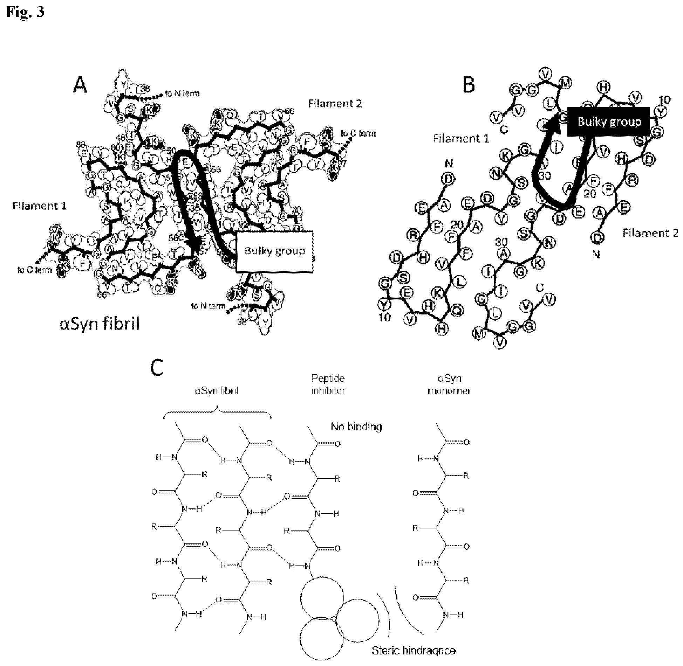

Our data show that peptides comprising two VATVA (SEQ ID NO. 4) sequences connected via a 5 amino acid linker containing 3 lysines efficiently inhibit αSyn amyloid fibril growth (Fig 2). VATVA (SEQ ID NO. 4) motifs correspond to the region 52-56 of αSyn that is on the interface of two filaments in amyloid fibril and are crucial for binding to the fibril end. Two copies of VATVA (SEQ ID NO. 4) sequence are necessary to interact with both filaments of the fibril via binding to corresponding regions of two αSyn molecules (Figure 3a,b).

Cyclization of peptides locks the conformation needed for binding and increases the inhibition activity. VATVA (SEQ ID NO. 4) sequence is rich in β-branched amino acids that possess high propensity to form β-sheets. Modification of the VATVA (SEQ ID NO. 4) sequence by changing A,V or T to other β-branched amino acids (V, T, I) or alanines leads to peptides that show lower but still sufficient ability to block the elongation of the amyloid fibrils.

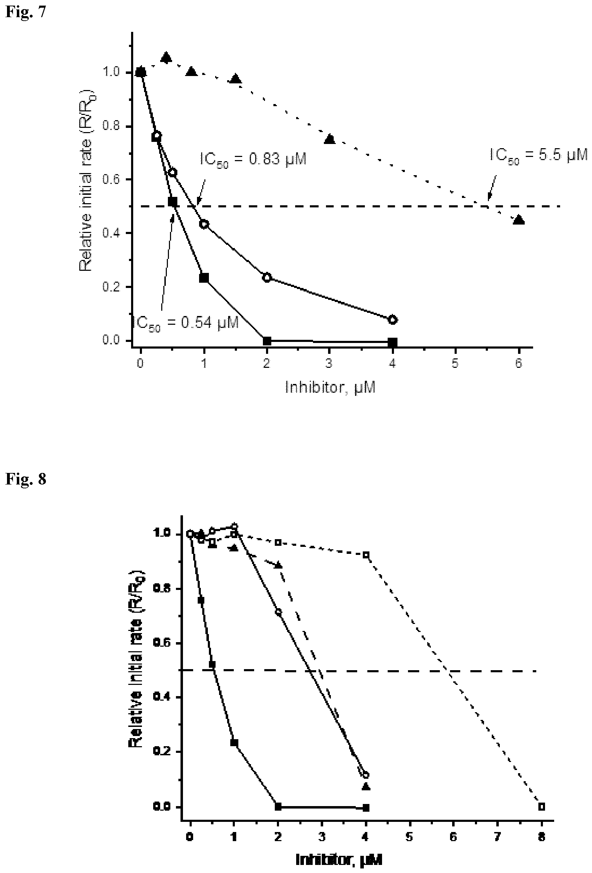

The optimal linker between two VATVA (SEQ ID NO. 4) groups is KKK[W or H]G (SEQ ID NO. 5) that has the length close to the distance between two (52-56) regions in αSyn fibril and contain three charged lysines to increase the solubility. However, it can be shortened to KKK with 1.5-fold decrease in activity (example 5, Fig 7) while removing charges decreases the activity about 10-fold.

Additional 2-4 lysines and arginines residues on C and N termini of disclosed peptides increase the positive charge of the molecule increasing its solubility and decreasing propensity to self-aggregate.

Bulky moiety can be attached to the peptide N-terminus via carboxylic group. Different groups including carboxyfluorescein, Ph₃C-CH₂-COOH, Ph₃P⁺(CH₂)₃-COOH applied as bulky moieties provide sufficient activity of resulted peptides. Meanwhile peptides modified on the N-terminus by acylation (CH₃COOH) shows much lower activity. It points to the importance of the size and non-planarity of the bulky moiety and not of the presence of particular functional groups in it.

Accordingly, the best activity was observed for the peptide (carboxyfluorescein)-K-C-K-VATVA-KKK-WG-VATVA-K-C-RRK (SEQ ID NO. 6), in which cysteins C and C were linked via m-xylene linker.

The disclosed active peptide inhibitors are cyclic peptides of 17-35 aa length comprising the sequence R¹-X¹-C-X²-X³X⁴X⁵X⁶X⁷-X⁸-KKK-X⁹-X¹⁰X¹¹X¹²X¹³X¹⁴-X¹⁵-C-X¹⁶ (SEQ ID NO. 1) wherein:
R¹ is a non-planar molecule selected from carboxyfluorescein residue, Ph₃C-CH₂-CO-, Ph₃P⁺(CH₂)₃-CO-, Ph₂N-(C₆H₄)-CO- and sulforhodamine residue, wherein R¹ is attached to the -NH- group of the N-terminal amino acid; and wherein R¹ provides steric hindrance for the amyloid fibril growth.
each of X³, X⁴, X⁵, X⁶, X⁷, X¹⁰, X¹¹, X¹², X¹³, X¹⁴ is independently selected from the amino acids V, I, A and T;
each of X², X⁸, X⁹ and X¹⁵ independently represents 0 to 2 amino acids;
each of X¹ and X¹⁶ independently represents 0 to 6 amino acids; and
wherein the two cysteines (C) are connected via a linker.

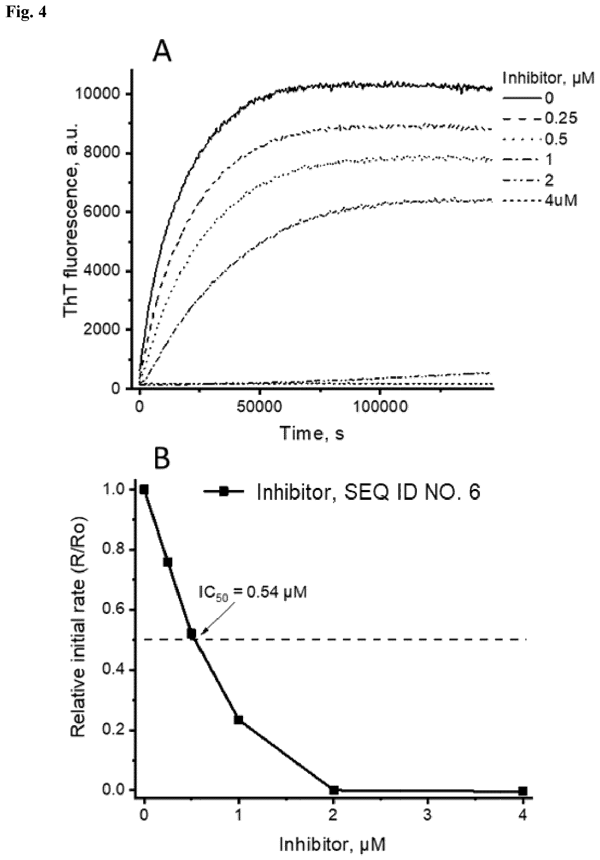

The interaction of the peptides of the invention with fibril ends and the mode of binding is supported by the relationship between the inhibitor concentration and the rate of the amyloid fibril elongation, which can be statisfactory described by the model assuming binding of one peptide molecule to the fibril end and clearly shows substoichiometric activtiy (Fig. 4).

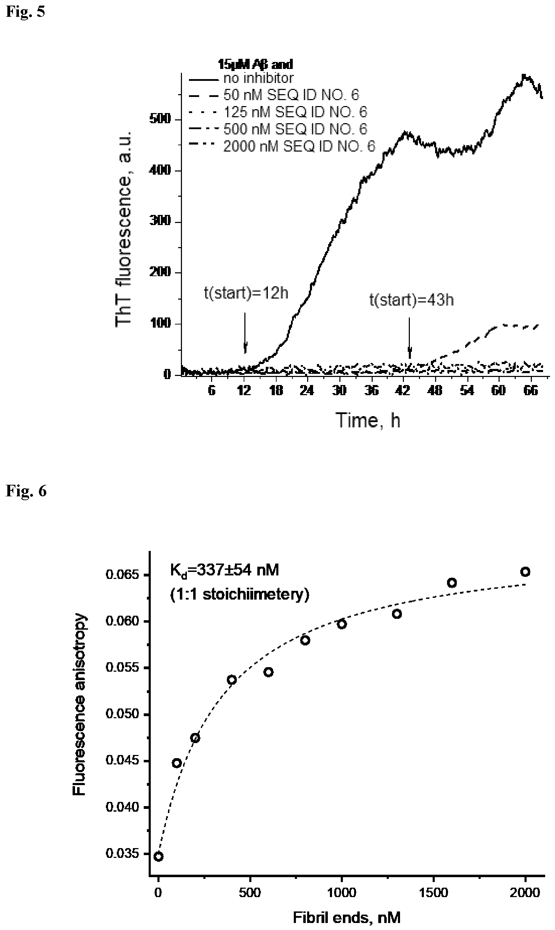

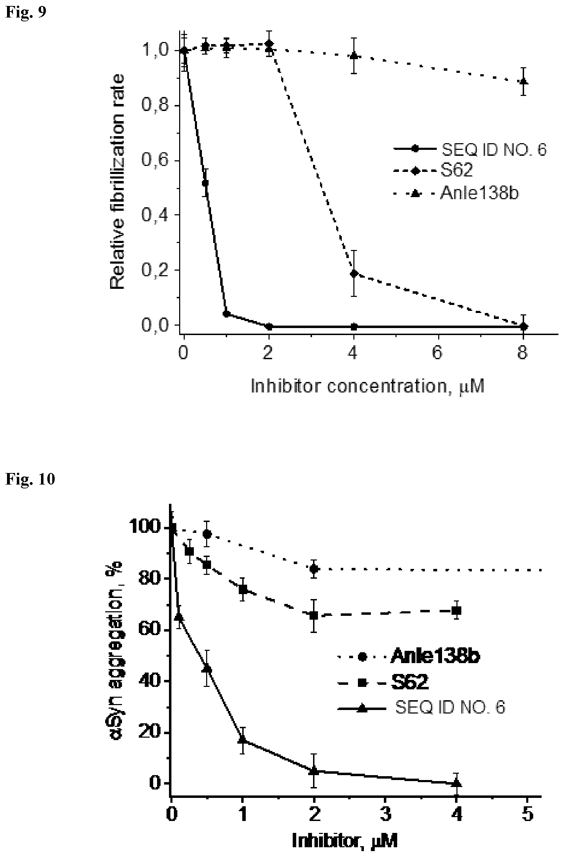

The inhibitory peptides retain their activity in the presence of excess of monomeric amyloidogenic protein or amyloid fibrils confirming that binding to the fibril end is stronger than the binding to monomer or to sides of fibril. Affinity of inhibitory peptide to the fibril end (~345 nM, Fig. 6) is close to the affinity of monomeric αSyn (300-500 nM) (Afitska et al., Biochim Biophys Acta, 2019, doi: 10.1016/j.bbapap.2019.05.003). The inhibitors are also able to prevent fibril elongation in a cellular environment (Fig. 10) that highlights their specificity to amyloid fibrils.

As compared to the prior art of peptides inhibiting αSyn or Aβ fibril growth we introduce following improvements:
- Introduction to inhibitory peptide of two copies of the sequence targeting separate monomers at the fibril end in order to cover both filaments by the inhibitory peptide. Namely, the most active peptide disclosed in this patent contains two copies of VATVA sequence that correspond to the region 52-56 of αSyn and allow to bind the interface of two filaments in amyloid fibril.
- Attachment of non-planar bulky group to the peptide N-terminus. After peptide binding to the end of fibril presence of such group makes the fibril end interface strongly non-planar preventing binding of monomeric protein (αSyn or Aβ) in its amyloid conformation. It also hinders binding of inhibitor molecules to each other in a fibril-like manner, decreasing the propensity of the inhibitor to aggregate and prevents inhibitor molecules from stacking at the end of the fibril. Introduction of such bulky group increases inhibition activity of peptide approximately 10-fold.
- Cyclization of peptide in order to lock in conformation optimal for the binding to the fibril end on the interface between filaments. This modification significantly improves inhibition activity of peptides targeting αSyn aggregation about 5-fold.
- The abovementioned improvements allowed to reach inhibition activity 5-10 times higher than any peptides or small molecules inhibiting amyloid fibril growth reported to date. Activity of the inhibitor peptides are crucial for their therapeutical application as it is problematic to deliver substantial amount of peptide-based drugs to the brain through BBB.

### Brief Description of the Drawings

**Figure 1****.** Schematic representation of the fibril growth inhibition by blocking amyloid fibril ends. The inhibitory peptides bind to the interface between two fibril filaments stopping further monomer attachment.
**Figure 2****.** Description of the design of the developed peptides that target αSyn fibril ends.
**Figure 3****.** Schematic representation of binding of the peptide with a bulky group to A) the interface between αSyn fibril filaments, B) aβ fibrils, and C) corresponding mechanism of action of the bulky group.
**Figure 4.** A) Kinetc curves of seeded αSyn aggregaton (50 µM αSyn, 500 nM seeds) at different concentrations of inhibitor SEQ ID NO. 6 (0 to 4 µM). B) Relationship between the relative initial rate of the ThT signal growth and the concentration of inhibitor corresponding to the curves presented in the panel A.
**Figure 5****.** Kinetics of fibrillization of aβ1-42 (15 µM) in the absence and in the presence of different concentrations of the inhibitors measured using ThT kinetic assay.
**Figure 6****.** Titration of 20 nM SEQ ID NO. 6 with sonicated αSyn asmyloid fibrils monitored via changes in fluorescence anisotropy of carboxyfluoresceine at the inhibitor. Concentration of fibril ends calculated at 1/100 of the concentration of fibrillized prtotein. The dahed line is the fitting curve according to the binding equation with Kd=337 nM and stoihiometry 1 molecule of inhibitor per fibril end.
**Figure 7****.** Effect of the sequence between two VATVA (SEQ ID NO. 4) motifs on the inhbition efficacy. Dependence of the relative initial rate of amyloid fibril growth on the inhibitor concentration measured for SEQ ID NO. 6 (closed squares), SEQ ID NO. 12 (open circles), sequence corresponding to SEQ ID NO. 6 wherein the sequence KKK between the two VATVA (SEQ ID NO. 4) motifs is replaced by polyethyleneglycol (PEG) (closed triangles). Relative initial rate (R/R₀) is the ratio of the rates on the increase of ThT signal during the first 2 hours of fibrillization of 50 µM αSyn in the presence of 500 nM seeds for samples with given concentration of inhbitor to the rate of increase without inhibitor. IC₅₀ were calculated as inhibitor concentration at which the rate decreases two-fold.
**Figure 8****.** Effect of bulky group on the peptide inhibition efficacy. Dependence of the relative initial rate of amyloid fibril growth on the inhibitor concentration measured for SEQ ID NO. 6 (closed squares), SEQ ID NO. 14 (open circles), SEQ ID NO. 13 (closed triangles), SEQ ID NO. 15 (open squares).
**Figure 9****.** Comparison of activity of SEQ ID NO. 6 and two best reported inhibitors (S62 and anle138b) in solution. Concentrations of αSyn and seeds were 50 and 0.5 µM, respectively. Error bars are SD of four repeats. Relative initial fibril growth rate as a function of inhibitor concentration, as compared to
**Figure 10****.** Comparison of activity of SEQ ID NO. 6 and two best reported inhibitors (S62 and anle138b) in cells using FRETAg assay. The relative amount of aggregated αSyn calculated from FRET signal. Each point is an average of 18 repeats from 3 experiments (independent cell culture preparations, different batches of PFFs), error bars are SD.

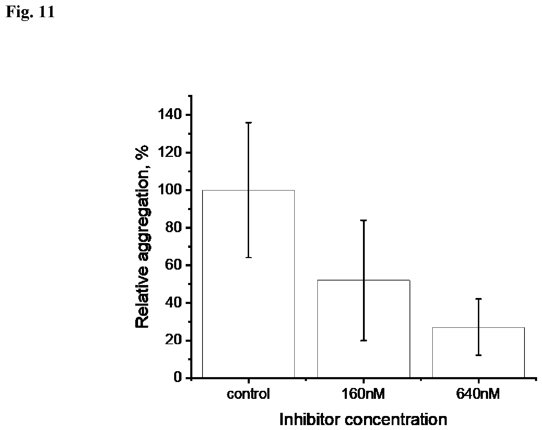

**Figure 11****.** Treatment of C. *elegans* with different concentrations of SEQ ID NO. 6 result in inhibition of fibril growth.

### Examples

### Abbreviations

AD - Alzheimer's disease
Aβ - amyloid beta
αSyn - alpha-synuclein
BBB- blood-brain barrier
DMSO -dimethyl sulfoxide
DMEM - Dulbecco's modification of Eagle's medium
FRET - Fluorescence resonance energy transfer
GFP - green fluorescent protein
PD - Parkinson's disease
PFF - preformed fibrils
PEG - polyethylene glycol
ThT - Thioflavin T
WT - wild type

### Example 1: Peptide design

The sequence of peptides targeting αSyn fibril ends was constructed in the following way:
We used the sequence VATVA (SEQ ID NO. 4) that is a part of αSyn sequence (aa 52-56) corresponding to the interface between two filaments in the fibril according to the structure of the most abundant polymorph (Li et al, Nat Commun, 2018, doi: 10.1038/s41467-018-05971-2). Two copies of such sequence were connected via a 5 amino-acid linker to allow simultaneous binding to the corresponding amino acids of two molecules of αSyn on the fibril end (Figure 2). To increase the inhibitory peptide solubility, we used 3 lysines in the linker while two other amino acids in the linker were aromatic tryptophan similar to αSyn residue H50 and glycine copying G51 of αSyn immediately preceding the VATVA (SEQ ID NO. 4) sequence (Figure 3a).

To keep the peptide in the desirable bended conformation, we cyclized it by introducing two cysteines on N and C termini and crosslinking them with m-xylene dibromide. To further improve the peptide solubility additional lysines or arginines were added to C and N terminus. Finally, bulky group was introduced at the N-terminus.

According to our observations VATVA (SEQ ID NO. 4) sequence of the peptide can be modified via replacement of amino acids to alanines or β-branched aliphatic amino acids (Thr, Val, Ile) without significant changes in activity (for example changing both sequences VATVA (SEQ ID NO. 4) to IATIA (SEQ ID NO. 7) or VAVVA (SEQ ID NO. 8) leads to less 2-fold change in activity, Table 2). The linker between two recognizing sequences (VATVA (SEQ ID NO. 4) or analogues) can be shortened to 3 amino acids with small changes in the activity (i.e. SEQ ID NO. 6 vs SEQ ID NO. 12). It can be also changed to PEG.

| Name | Structure | IC₅₀, µM |
|---|---|---|
| SEQ **ID** NO. 6 | fl -KCKVATVAKKKWGVATVAKCRRK, m-xylene bridge | 0.5 |
| SEQ **ID** NO. 9 | fl -KCKVAVVAKKKWGVAVVAKCRRK, m-xylene bridge | 3.5 |
| SEQ ID NO. 10 | fl -KCKAVTAVKKKWGAVTAVKCRRK, m-xylene bridge | 3.2 |
| SEQ ID NO. 11 | fl -KCKIATIAKKKWGIATIAKCRRK, m-xylene bridge | 1.2 |

| | | |
|---|---|---|
| fl stands for N-terminal modifications with 5(6)carboxyfluorescein, Highlighted amino acids represents the cyclization cysteines. | | |

### Example 2: Peptides of the invention inhibit αSyn fibril growth

The peptides were tested in seeded ThT-based kinetic assay of αSyn aggregation as described in materials and methods section.

The obtained data showed that the developed peptides are potent inhibitors of αSyn fibril growth, with IC₅₀ reaching as low as 0.5 µM (Fig. 4).

There is a hyperbolic relationship between the inhibitor concentration and initial fibril growth rate that is in line with proposed model of action.

R/Ro ≈ IC₅₀/(IC₅₀+C(inhibitor)), where R and Ro is the initial fibril growth rate in the presence of given inhibitor concentration and without inhibitor, respectively.

Meanwhile at higher concentrations of inhibitor there is almost complete stop of fibril growth.

### Example 3: Peptides inhibit Aβ fibril growth

The fibril binding sequence of the developed peptides is capable of recognizing other amyloid fibrils such as fibrils of Aβ.

The peptides were tested in non-seeded ThT kinetic aggregation assay. For that the peptides were mixed with 15 µM aβ(1-42) and then the fibrillization was assessed based on the increase of ThT fluorescence intensity.

The peptides were capable to severely inhibit amyloid beta fibril formation even at the lowest tested concentration (50 nM), postponing the beginning of the exponential growth phase (also known as 'lag time') of the aggregation from 12h to 43h that corresponds to about 3.5 times slowdown of the aggregation (Fig. 5).

### Example 4: Developed peptides binds to the ends of αSyn fibrils with affinity ≈350 nM.

To check affinity of the peptide SEQ ID NO. 6 that contain 5(6)carboxyfluoresceine at the N-terminus to the ends of αSyn fibrils it was titrated sonicated pre-formed αSyn fibrils (PFFs) and binding was monitored via anisotropy of 5(6)carboxyfluoresceine fluorescence. The concentration of fibril ends was estimated as 1/100 of the concentration of fibrillized protein based on the average length of seeds (~50 nm) and the distance between two monomers in fibril (~0.5 nm). The binding of the peptide to the fibril ends corresponds to Kd=337±54 nM and stoichiometry 1:1 (Fig 6). Meanwhile, analogues experiments show no detectable interactions with monomeric αSyn or with long αSyn fibrils pointing that the interaction of SEQ ID NO. 6 with monomeric αSyn and with the sides of αSyn fibrils is negligible comparing to its affinity to the fibril ends.

### Example 5: The length of the linker affects inhibition activity

Shortening of the linker between two VATVA (SEQ IN NO. 4) binding sequences to three lysines decreases the inhibitory activity approximately 1.5 fold (Fig 7). Meanwhile substituting 3 lysines by PEG linker of the same length (NH₂-CH₂-CH₂-O-CH₂-CH₂-O-CH₂-COOH) while keeping WG amino acids leads to ~10 fold decrease in activity. It points out that for optimal activity the linker between two VATVA (SEQ IN NO. 4) groups should be at least 3 aa long and should bear positive charge.

| Name | Structure | IC₅₀, µM |
|---|---|---|
| SEQ **ID** NO. 6 | fl -KCKVATVAKKKWGVATVAKCRRK, m-xylene bridge | 0.5 |
| SEQ ID NO. 12 | fl -KCKVATVAKKKVATVAKCRRK, disulfide bridge | 0.8 |

| | | |
|---|---|---|
| fl stands for N-terminal modifications with 5(6)carboxyfluorescein, Highlighted amino acids represents the cyclization cysteines. | | |

### Example 6: Introduction of a bulky group to the peptides increases their inhibition activity

Presence of bulky group strongly increases the inhibition activity of peptide, but the group itself can belong to different classes of compound, pointing that the key property is the size and not particular chemical structure. We compared inhibition activity of three peptides of the same sequence differing only by N-terminal bulky group. The best inhibition activity was observed for peptide modified with 5(6) carboxyfluorescein. Other bulky groups, namely derivatives of triphenilmethane or triphenylphosphonium connected by flexible linker yielded peptides about 6 fold less active as inhibitors. Meanwhile the activity of peptide without bulky group was 12 times lower (Fig 8)

| Name | Structure | IC₅₀, µM |
|---|---|---|
| **SEQ ID NO. 6** | **fl -KCKVATVAKKKWGVATVAKCRRK, m-xylene bridge** | 0.5 |
| **SEQ ID NO. 13** | Ph3C-KCKVATVAKKKWGVATVAKCRRK, disulfide bridge | 2.8 |
| **SEQ ID NO. 14** | Phos-KCKVATVAKKKWGVATVAKCRRK, disulfide bridge | 3 |
| **SEQ ID NO. 15** | ac-WGKCVATVAKKKHGVATVACE, disulfide bridge | 6.1 |

| | | |
|---|---|---|
| fl stands for N-terminal modifications with 5(6)carboxyfluorescein, ac stands for N-terminal acylation, Ph₃C stands for N-teminal acylation with triphenylmethane derivative Ph₃C-CH₂-COOH, Phos stands for N-teminal acylation with triphenylphosphonium derivative Ph₃P⁺-CH₂- CH₂- CH₂-COOH, Highlighted amino acids represents the cyclization cysteines. | | |

### Example 7: Cyclization of peptides increases their inhibition activity

Cyclization of peptides strongly increase the activity, as can be seen in case of SEQ ID NO. 6 (cyclized) and SEQ ID NO. 18 (not cyclized, comparative example). However, the linker used for cyclization does not significantly influence the inhibitor activity. Namely, peptide with disulfide bridge (SEQ ID NO. 16) show the same activity in vitro as the same peptide with m-xylene crosslink between the cysteines (SEQ ID NO. 17). Importantly, non-cyclized peptide (SEQ ID NO. 18) suffers more than 4-fold activity decrease as compared to the cyclic analogues (SEQ ID NO. 16 and SEQ ID NO. 17).

| Name | Structure | IC₅₀, µM |
|---|---|---|
| SEQ **ID** NO. 6 | fl -KCKVATVAKKKWGVATVAKCRRK, m-xylene bridge | 0.5 |
| SEQ ID NO. 16 | fl -GKCVATVAKKKHGVATVACE, disulfide bridge | 2 |
| SEQ ID NO. 17 | fl-GKCVATVAKKKHGVATVACE, m-xylene bridge | 1.7 |
| SEQ ID NO. 18 | fl -GKGVATVAKKKHGVATVAE | 10 |

| | | |
|---|---|---|
| fl stands for N-terminal modifications with 5(6)carboxyfluorescein, Highlighted amino acids represents the cyclization cysteines. | | |

### Example 8: The peptides inhibit amyloid fibril growth in cells

To test the activity of the peptide inhibitors in cells we used FRETAg assay - a cell-based method that directly report the amount of formed fibrils and does not interfere with the aggregation kinetics (Galkin et al., J Neurochem, 2021, doi: 10.1111/jnc.15528). Additionally, FRETAg allows to use PFF to induce αSyn aggregation in cells, making the aggregation process controllable and reproducible.

FRETAg uses a pair of αSyn monomers labeled with Atto565 and Atto647 fluorophores. When αSyn is fibrillar, the close proximity of the labels (<5 nm) enables FRET, with excitation of Atto565 resulting in emission from Atto647. Thus, the appearance of a FRET signal indicates the formation of αSyn fibrils, distinguishing them from monomers. Labeled αSyn is prepared in vitro and added to cells during the assay, where extracellular monomeric αSyn can effectively penetrate the plasma membrane and aggregate in cytoplasm.

Our data showed that the developed peptides are effective even at very low concentrations - at 100 nM concentration it was able to inhibit aggregation rate by 30%. When compared to other reported inhibitors, such as S62 or Anle138b, the peptides showed superior inhibition activity with the resulting IC₅₀ about 500 nM. Noteworthy, that the peptides were able to completely stop αSyn aggregation at higher concentration, indicating their specificity and efficacy inside cells (Fig. 10).

### Example 9: The peptides can inhibit fibril growth in C. elegans

To test the activity of peptides *in vivo* we used UM0011 strain that express hWT αSyn without any fluorescent protein tags, as our fibril-binding sequence is specific towards WT fibrils, and protein-fusion can significantly change the inhibitor binding. The experimental design comprised treatment of animals with preformed αSyn fibrils for them to be incorporated in the tissues and then these fibrils can elongate upon interaction with expressed αSyn in those tissues.

We found that C. *elegans* treated with the peptide have lower amount of fibrils capable of aggregation seeding and the decrease is dose-dependent (Fig. 11).

### Peptide synthesis

The synthesis was performed at a 0.1-mmol scale using standard sidechain protected fluorenylmethoxycarbonyl (Fmoc)-amino acids and HBTU/HOBt coupling protocol. Rink amide resin (0.44 mmol/g reactive group concentrations) was used as solid support. At the end of the synthesis, peptidylresin was isolated and washed twice by NMP.

Two equivalents (0.15 mmol) of the carboxy derivative of bulky group used for N-terminus capping (carboxyfluoresceine, etc) were dissolved in 1 mL of NMP mixed with two eq. of HBTU/HOBt coupling solution (in DMF) and added to Fmoc-deprotected peptidylresin (0.075 mmol) swelled in 1 mL of NMP. After a few minutes of shaking, five eq. of DIEA solution was added. Then, the reaction mixture was stirred overnight at 40 °C. Resin was filtrated and washed with NMP, methanol and DCM.

Cleavage and deprotection of the peptidylresin were performed for 2 h using a 10 mL trifluoro-acetic acid (TFA) solution containing water (5%, v/v), triisopropilsilane (iPr₃SiH, 2.5%, v/v), phenol (1%, w/v), thioanisole (5%, v/v) and ethanedithiol (2.5%, v/v). The solution was concentrated in vacuo and the peptide was precipitated by using ice-cold diethyl ether and then pelleted by centrifugation. The pellet was washed with diethyl ether and dried. The peptides were solubilized with aqueous TFA (0.05 %, v/v). HPLC purification was carried out on a C8 column in water/acetonitrile mixture containing 0.05% TFA with linear gradients depending on the peptide (typically 10 to 60 % of acetonitrile for 30 min) and monitored at 210 nm (detection of peptide bond) and 280 nm (for detection of capping groups or tryptophanes). Prior to use, peptides were dissolved in phosphate buffer, aliquoted and stored at -20 °C. Concentrations of the peptides were determined based on light absorbance. For peptides modified with carboxyfluoresceine was used absorbanece coefficient ε = 70000 M⁻¹ cm⁻¹ at 480 nm, for ones containing no Fluoresceine but containing Trp residues was used its absorbance at 280nm (ε = 5600 M⁻¹ cm⁻¹).

### α-Synuclein (αSyn) expression and purification

Expression and purification of αSyn and its A53T mutant were performed as described previously (Afitska et al., Biophys J, 2017, doi: 10.1016/j.bpj.2017.08.027). Shortly, αSyn was expressed in E. coli, purified by ion-exchange FPLC and stored in 10 mM Tris-HCl buffer, pH 7.4 at -20°C at 200 µM concentration. The concentration of αSyn monomer was determined using Tyr absorption at 275 nm.

### Amyloid beta expression and purification

The recombinant production and purification of monomeric Amyloid-β(1-42) from E. coli were performed as described previously (Hoarau, PLos One, 2016, doi: 10.1371/journal.pone.0161209). The concentration was determined using Tyr absorption at 275 nm considering correction for Phe residues.

### Preparation of αSyn fibrils and PFF

αSyn amyloid fibrils were formed by mixing 1 µM αSyn PFF (pre-formed fibrils) with 100 µM αSyn monomer in buffer containing 10 mM Tris-HCl, pH 7.4, and 130 mM NaCl. The resulting solution was incubated in a thermoshaker for 24 h under constant agitation. αSyn fibrils for preparation of initial PFF were obtained in non-seeded aggregation of 100 µM αSyn for 5 days under the same conditions.

PFF were prepared by sonication of αSyn fibrils. An Eppendorf tube with 50-200 µl of 50 µM fibril solution was immersed in a low power bath sonicator (Sonorex Digitec DT 100) for 30 min. The activity of PFF was measured in a ThT-based in vitro aggregation assay (Kyriukha et al., J Med Chem, 2019, doi: 10.1021/acs.jmedchem.9b01400). Briefly, PFF were mixed with 50 µM αSyn monomer and the fibrillization rate was measured by the increase in ThT fluorescence.

### αSyn labeling

αSyn-A18C mutant was labeled with Atto565, Atto647 maleimides (Atto-Tec, Germany, cat. no. AD 565 and AD 647) or Sulforhodamine-B maleimide (prepared as described in (Kyriukha et al., J Med Chem, 2019, doi: 10.1021/acs.jmedchem.9b01400)). Before labeling, the protein was incubated overnight with a twofold molar excess of tris(2-carboxyethyl)phosphine (TCEP, from TCI, cat. no. T1656) to reduce any disulfide bonds formed. Then, the protein solution (200 µl of 185 µM in 10 mM Tris-HCl, pH 7.4 buffer) was mixed with 1.5 equiv. of the fluorescent label (as a solution in DMSO). The solution was further shaken for 2 h at 4°C (low temperature was used to minimize αSyn aggregation). After that an additional 0.5 equiv. of the label were added, and the reaction mixture was then incubated overnight at 4°C with gentle shaking. The excess of the unreacted label was removed using a Zeba Spin desalting column, 7 k molecular weight cut-off (Thermo Fisher Scientific, cat. no. 89882). Covalent conjugation of protein with a fluorescent label was confirmed by ESI mass spectrometry. The concentration of labeled protein was determined by absorbance of the fluorescent label (using ε = 90 000 M⁻¹cm⁻¹ at 565 nm for Sulforhodamine-B, ε = 120 000 M⁻¹cm⁻¹ at 565 for Atto565 and at 647 nm for Atto647). The resulting solution of labeled protein was protected from light and stored at -20°C.

### Thioflavin T assessment of αSyn aggregation

To start the aggregation, monomeric αSyn was mixed with buffer and seeds. This solution was immediately aliquoted to the Eppendorf tubes containing inhibitor solutions, including the control tube without inhibitor. This procedure was repeated for each tested inhibitor. Prepared samples were pipetted to the plate (four repeats for each concentration of each tested inhibitor), and the fluorescence intensity was measured as described above.

Typically for measurements of IC₅₀ values of 50 µM αSyn and 500 nM (concentration of protein in the form of short sonicated fibrils), seeds were used. For each inhibitor, IC₅₀ measurements were performed as three to eight independent experiments with different batches of seeds.

ThT fluorescence signal intensity is linearly proportional to the fibril concentration, therefore the fibril growth rate can be calculated from its increase. Fibril growth rates were calculated for each well separately and then the average values of the relative fibril growth rates were calculated in groups of parallel samples. The relative fibril growth rates were calculated as the ratio of the fibril growth rate in the presence and in the absence of the inhibitor. Therefore, the relative fibril growth rate of αSyn in the absence of inhibitor was 1.

The obtained relative fibril growth rates were plotted as a function of inhibitor concentration. IC₅₀ was determined as the concentration of inhibitor that led to a 2 fold decrease in the fibril growth rate (where the relative fibril growth rate was 0.5 of the initial value). SD of the relative rate was calculated as a sum of relative SD for samples at a given inhibitor concentration and relative SD for samples without an inhibitor.

### Thioflavin T assessment of amyloid beta aggregation

Thioflavin T (ThT)-based Aβ aggregation kinetics assays were performed similarly to ones for αSyn but 15 µM Aβ1-42 monomer was used and we did our best to minimize the time between the mixing and start of the measurements.

### Assessment of αSyn aggregation inhibitors in cells (FRETAg)

HeLa Kyoto cells were obtained from the Cell facility of the Institute of Organic Chemistry and Biochemistry (Prague, Czech Republic). Cells were cultured in the complete growth medium, which contained Dulbecco's modification of Eagle's medium (DMEM, Sigma-Aldrich, cat. no. D6046), 10% fetal bovine serum (FBS, Sigma-Aldrich, cat. no. F7524), 200 mM L-glutamine (GlutaMAX, Gibco, cat. no. 35050061), 0.2% Primocin (InvivoGen, cat. no. ant-pm-1) and maintained in a humidified atmosphere containing 5% CO₂ at 37 °C in T25 flasks.

To evaluate αSyn aggregation in cells HeLa cells were plated on a 96-well plate (Greiner CellStar, Sigma-Aldrich, cat. no. M0812) 24 h before the experiment. The cells were treated as follows. To all wells, except negative control, 1 µM PFF were added. After 24 h cells were washed with complete medium and then incubation was continued in αSyn-free medium for 6 h to remove residual PFF. After that, 400 nM of labeled αSyn (1:1 mixture of αSyn-A18C-Atto565 and αSyn-A18C-Atto647). If necessary, the selected inhibitors were added just directly after αSyn addition. After 24 h of incubation, cells were washed twice and additionally incubated in a complete medium overnight. To eliminate background fluorescence during measurements, cell medium was changed to Ringer solution (136 mM NaCl, 4.7 mM KCl, 1 mM CaCl₂, 1 mM MgCl₂, 5mM NaH₂PO₄, 20 mM HEPES, 10 mM Glucose). The fluorescence of formed amyloid fibrils was measured using Tecan Spark microplate reader (550 nm excitation, 650 nm emission, slits 20 nm). The relative amount of aggregated protein (Fᵣₑₗ) at a given inhibitor concentration was calculated as the ratio of the fluorescence signal (Sᵢ) to the signal in the absence of an inhibitor (maximal possible fibrillization, Smax), both after subtraction of background (signal in the absence of seeds, S₀): Fᵣₑₗ=(Sᵢ-S₀)/(Sₘₐₓ-S₀).

In each experiment we used 12 repeats (wells) for controls (S₀ and Sₘₐₓ) and 6 repeats for each concentration point. The relative amount of aggregated protein was fitted to a single-site binding model using Origin2015 software.

### Assessment of αSyn aggregation inhibitors in C. elegans

C. elegans strain UM0011 was kindly provided by Prof. Garry Wong (Honk Kong). This strain express hWT αSyn under pan-neuronal promotor and GFP in dopaminergic neurons.

Cultivation and maintenance of C. elegans strains followed conventional protocols. Synchronization of the worms was achieved through hypochlorite bleaching, and subsequently, they were allowed to hatch overnight at a temperature of 16 °C in M9 media. Following this, the worms were cultured on nematode growth medium plates that were seeded with OP50 E. coli. Then worms were removed from plates with M9 buffer, and a 30% sucrose solution was used to remove dead animals and bacteria. Afterwards worms were resuspended in M9 buffer supplemented with 500 nM αSyn PFF and kept on orbital shaker. After 3h incubation worms were washed with M9 buffer 3 times and then resuspended in M9 buffer supplemented with OP50 and different SEQ ID NO. 6 inhibitor concentrations (0, 160 nM, 640 nM). After 24 h worms were washed with M9 buffer 3 times and then the worms were lysed using freeze-thawing and sonicator bath for 30 minutes. Lysate was centrifuged at 10,000g for 10 minutes. Then it was mixed with 100 µM αSyn monomer and used in ThT-based assay to evaluate the resulting amount and activity of fibrils formed in C. elegans with and without inhibitor.

### Industrial applicability

The present invention provides peptides that are aimed to be used as drugs to stop or slow down the progression of Parkinson's or Alzheimer's diseases.

## Claims

1. A cyclic peptide containing 17 to 35 amino acids having an amino acid sequence of general structure R¹-X¹-C-X²-X³X⁴X⁵X⁶X⁷-X⁸-KKK-X⁹-X¹⁰X¹¹X¹²X¹³X¹⁴-X¹⁵-C-X¹⁶ (SEQ ID NO. 1),
wherein
R¹ is selected from carboxyfluorescein residue, Ph₃C-CH₂-CO-, Ph₃P⁺(CH₂)₃-CO-, Ph₂N-(C₆H₄)-CO- and sulforhodamine residue, wherein R¹ is attached to the -NH- group of the N-terminal amino acid;
each of X³, X⁴, X⁵, X⁶, X⁷, X¹⁰, X¹¹, X¹², X¹³, X¹⁴ is independently selected from the amino acids V, I, A and T;
each of X², X⁸, X⁹ and X¹⁵ independently represents 0 to 2 amino acids;
each of X¹ and X¹⁶ independently represents 0 to 6 amino acids; and
wherein the two cysteines (C) are connected via a linker, thus forming a cycle.

2. A cyclic peptide according to claim 1 wherein:
X¹ is selected from K, KK, GK and WGK; and/or
X² is K or is not present; and/or
X⁸ is not present; and/or
X⁹ is WG, HG or is not present; and/or
X¹⁵ is K or is not present; and/or
X¹⁶ is selected from K, E, RK, RKK, RRK, KRRK (SEQ ID NO. 19).

3. A cyclic peptide according to claim 1 or 2 having an amino acid sequence of the general structure R¹-X¹-C-X²-VATVA-X⁸-KKK-X⁹-VATVA-X¹⁵-C-X¹⁶ (SEQ ID NO. 2).

4. A cyclic peptide according to claim 1 having an amino acid sequence of the general structure R¹-K-C-K-VATVA-KKK-WG-VATVA-K-C-RRK (SEQ ID NO. 3).

5. A cyclic peptide according to any one of claims 1 to 4, wherein the linker connecting the two cysteins is selected from disulfide bond, -CH₂-(C₆H₄)-CH₂-, -(CH₂)ₙ- wherein n=2-6, -CH₂CH₂OCH₂CH₂-, and bismaleimide linker.

6. A cyclic peptide according to claim 1 having the formula (carboxyfluorescein)-K-C-K-VATVA-KKK-WG-VATVA-K-C-RRK (SEQ ID NO. 6), wherein the linker connecting the two cysteins is m-xylene linker.

7. A cyclic peptide according to any one of claims 1 to 6 for use as a medicament.

8. A cyclic peptide according to any one of claims 1 to 6 for use in the prevention or treatment of a disorder selected from Parkinson's disease and Alzheimer's disease.

9. A pharmaceutical composition comprising as an active ingredient at least one peptide according to any one of claims 1 to 6 and at least one pharmaceutically acceptable excipient.

10. An *in vitro* method for reducing or inhibiting aggregation of αSyn, Aβ, or their mutants, comprising a step of contacting amyloid fibrils with a peptide according to any one of claims 1 to 6.
